# EUROPEAN PATENT SPECIFICATION

(11) **EP 2 968 672 B1**
(45) Date of publication and mention of the grant of the patent: **06.02.2019**
(21) Application number: 14725270.4
(22) Date of filing: 13.03.2014
(51) Int. Cl.: A61L 27/36, A61L 27/38, C12N 5/00, C12N 5/07

(54) **USE OF MICROPARTICLES AND ENDOTHELIAL CELLS WITH DECELLULARIZED ORGANS AND TISSUES**
VERWENDUNG VON MIKROPARTIKELN UND ENDOTHELZELLEN MIT DEZELLULARISIERTEN ORGANEN UND GEWEBEN
UTILISATION DE MICROPARTICULES ET DE CELLULES ENDOTHÉLIALES DANS DES ORGANES ET DES TISSUS DÉCELLULARISÉS

(30) Priority: 15.03.2013 US 201361790118 P
(43) Date of publication of application: 20.01.2016
(73) Proprietor: Miromatrix Medical Inc., Eden Prairie, Minnesota 55347 (US)
(72) Inventor: ROSS, Jeffrey, Chaska, Minnesota 55318 (US); SEETAPUN, Dominique, Minnetonka, Minnesota 55345 (US)
(74) Representative: Uexküll & Stolberg
(86) International application number: PCT/US2014/026456
(87) International publication number: WO 2014/168719

(56) References cited:
- WO-A1-2012/005760
- BASAK E UYGUN ET AL: "Organ reengineering through development of a transplantable recellularized liver graft using decellularized liver matrix", NATURE MEDICINE, vol. 16, no. 7, 1 July 2010 (2010-07-01), pages 814-820, XP55069907, ISSN: 1078-8956, DOI: 10.1038/nm.2170
- S. M. JAY ET AL: "Engineering of multifunctional gels integrating highly efficient growth factor delivery with endothelial cell transplantation", THE FASEB JOURNAL, vol. 22, no. 8, 16 April 2008 (2008-04-16) , pages 2949-2956, XP055130471, ISSN: 0892-6638, DOI: 10.1096/fj.08-108803
- SHIMIZU ET AL: "Effective cell-seeding technique using magnetite nanoparticles and magnetic force onto decellularized blood vessels for vascular tissue engineering", JOURNAL OF BIOSCIENCE AND BIOENGINEERING, ELSEVIER, AMSTERDAM, NL, vol. 103, no. 5, 1 May 2007 (2007-05-01), pages 472-478, XP022136689, ISSN: 1389-1723, DOI: 10.1263/JBB.103.472
- STEPHEN F. BADYLAK ET AL: "Whole-Organ Tissue Engineering: Decellularization and Recellularization of Three-Dimensional Matrix Scaffolds", ANNUAL REVIEW OF BIOMEDICAL ENGINEERING, vol. 13, no. 1, 15 August 2011 (2011-08-15), pages 27-53, XP55127197, ISSN: 1523-9829, DOI: 10.1146/annurev-bioeng-071910-124743
- T. H. PETERSEN ET AL: "Tissue-Engineered Lungs for in Vivo Implantation", SCIENCE, vol. 329, no. 5991, 30 July 2010 (2010-07-30), pages 538-541, XP55126477, ISSN: 0036-8075, DOI: 10.1126/science.1189345
- TABATA YASUHIKO ET AL: "Neovascularization effect of biodegradable gelatin microspheres incorporating basic fibroblast growth factor", JOURNAL OF BIOMATERIALS SCIENCE. POLYMER EDITION, VSP, UTRECHT, NL, vol. 10, no. 1, 1 January 1999 (1999-01-01), pages 79-94, XP009091222, ISSN: 0920-5063
- MICHAEL LOVETT ET AL: "Vascularization Strategies for Tissue Engineering", TISSUE ENGINEERING PART B: REVIEWS, vol. 15, no. 3, 1 September 2009 (2009-09-01), pages 353-370, XP055129909, ISSN: 1937-3368, DOI: 10.1089/ten.teb.2009.0085

## Description

### Background

Tissue engineering is a rapidly growing field that seeks to repair or regenerate damaged or diseased tissues and organs through the implantation of combinations of cells, scaffolds and soluble mediators. Current stem cell differentiation and primary cell culture is generally achieved under 2-dimentional (2D) culture conditions. That system allows for the expansion of specific cell populations but is limited in its ability retain functional cellular phenotypes, to support high density cell culture and long term primary or differentiated cell function. For example, in contrast to the limited availability of large numbers of primary cells needed for certain cellular therapies, the number of stem cells can be greatly expanded while retaining the ability to differentiate into specific lineages. The control of stem cell fate (e.g., differentiation), either *in vivo* or *in vitro,* has been attributed principally to genetic and molecular mediators (e.g., growth factors and transcription factors). Although stem and progenitor cell differentiation can result in cells with appropriate lineage- or tissue-specific gene expression, the differentiated cells can lack functional properties needed for *in vitro* or *in vivo* applications.

Basak et al: "Organ reengineering through development of a transplantable recellularized liver graft using decellularized liver matrix", Nature Medicine, vol. 16, no. 7, 2010, pages 814-820 disclose a decellularized liver graft which has intact vasculature and is repopulated with hepatocytes and endothelial cells. WO 2012/005760 A1 discloses the use of perfusion decellularisation of organs to create decellularized ECM with intact vasculature and the subsequent repopulation with cells. Jay et al: "Engineering of multifunctional gels integrating highly efficient growth factor delivery with endothelial cell transplantation", The FASEB Journal, vol. 22, no. 8, 2008, pages 2949-2956 disclose a polymeric scaffold which is seeded with endothelial cells and alginate microparticles loaded with VEGF as artificial growth factor delivery systems. SHIMIZU et al: "Effective cell-seeding technique using magnetite nanoparticles and magnetic force onto decellularized blood vessels for vascular tissue engineering", JOURNAL OF BIOSCIENCE AND BIOENGINEERING, vol. 103, no. 5, 2007, pages 472-478 describe the use of magnetic force and magnetite nanoparticles for increasing the cell-seeding efficiency in tubular scaffolds derived from porcine decellularized common carotid artery.

### Summary of the Invention

The invention provides a method to maintain, reduce a decrease in or expand capillary vessel lumen diameter in a re-endothelialized and/or recellularized (with cells other than endothelial cells) extracellular matrix of a mammalian organ, tissue or portion thereof with an intact vascular bed, for example, to ensure proper capillary diameter to sustain continuous blood flow upon transplantation. The method includes providing or preparing a decellularized extracellular matrix of a mammalian organ, tissue or portion thereof with an intact vascular bed and providing or preparing a population of endothelial cells or stem or progenitor cells capable of differentiation into endothelial cells. An amount of the cells and an amount of a first aqueous solution comprising biocompatible nanoparticles or microparticles are introduced concurrently or sequentially to the decellularized extracellular matrix. The amount of the cells is effective to re-endothelialize the vasculature of the decellularized extracellular matrix and the amount of the nanoparticles or microparticles, when circulated through the vasculature, maintains, reduces a decrease, e.g., a decrease to about 4 microns, or expands, e.g., up to about 15 to about 20 microns, capillary vessel lumen diameter in the vasculature, during or after re-endothelialization relative to a corresponding re-endothelialized decellularized extracellular matrix that lacks the nanoparticles or microparticles. In one embodiment, the nanoparticles or microparticles are biodegradable. In one embodiment, the nanoparticles or microparticles are rapidly biodegradable upon an added agent or energy source. In one embodiment, the nanoparticles or microparticles are not biodegradable. The nanoparticles may be formed of any biocompatible material and may be of any shape that allows for passage through the vasculature. In one embodiment, the nanoparticles or microparticles are spherical or elliptical in shape. In one embodiment, the average diameter of the nanoparticles or microparticles is from about 0.5 µm to about 30 µm or about 5 µm to about 20 µm. In one embodiment, the nanoparticles or microparticles are deformable. In one embodiment, the nanoparticles or microparticles are formed of polymers, including naturally occurring and synthetic (non-naturally occurring) polymers. In one embodiment, the nanoparticles or microparticles are formed of protein and non-protein polymers. In one embodiment, the nanoparticles or microparticles are modified to include carboxylates, esters, amines, aldehydes, alcohols, or halides, as well as functional molecules such as ligands of magnetic molecules.. In one embodiment, an exterior energy source is added such as, but not limited to, light, magnetic, mechanical or ultrasound that degrades the particles. In one embodiment, the aqueous solution is added after re-endothelialization. In one embodiment, the nanoaprticles or micropraticles are removed by washing the re-endothelialized vasculature with another solution that lacks the particles and may contain an agent that degrades the particles. In one embodiment, the method includes introducing a second aqueous solution comprising biocompatible nanoparticles or microparticles having an average diameter that is at least 10% greater than the nanoparticles or microparticles in the first solution. The concentration of particles can be varied and in any amount that achieves the objective. For example, the first solution may include about 300 to about 50,000,000 particles per µL.

The decellularized extracellular matrix may be from any organ or tissue so long as the organ and tissue have an intact vascular (capillary) bed allowing for a circulation of a solution into a vascular conduit and out of a vascular conduit of the organ or tissue (an "intact" vasculature). In one embodiment, the decellularized organ is a decellularized a heart, a pancreas, a liver, a kidney, bone, or a lung. Any cell type that can re-endothelialize the extracellular matrix vasculature of the decellularized organ, tissue or portion thereof with an intact vasculature may be employed. For example, the cells may be obtained from iPS cells. In one embodiment, the cells are introduced to the matrix either by injection or perfusion, or a combination thereof. In one embodiment, the cells are introduced to the vasculature either by injection or perfusion, or a combination thereof. In one embodiment, the cells that are introduced to the decellularized extracellular matrix are primary cells. In one embodiment, the cells that are introduced to the decellularized extracellular matrix are a plurality of different cell types intended to fully or partially recellularize the organ, tissue, or portion of. In one embodiment, the cells and the perfusion decellularized organ, tissue or portion thereof are allogeneic.. In one embodiment, the cells and the perfusion decellularized organ, tissue or portion thereof are xenogeneic.

### Brief Description of the Figures

Figure 1A shows a photograph of a porcine liver that was perfusion decellularized.
Figures 1B-C show a scanning electron microscope (SEM) photograph of a vessel and the parenchymal matrix, respectively, of the perfusion decellularized porcine liver.
Figure 2 provides a gross view of an immersion decellularized rat liver, in which fraying of the matrix can be seen at both low (left) and high (right) magnification.
Figure 3 shows SEM photographs of immersion decellularized rat liver (A and B) and perfusion decellularized rat liver (C and D).
Figure 4 provides histology of immersion decellularized liver (A, H&E staining; B, Trichrome staining) and perfusion decellularized liver (C, H&E staining; D, Trichrome staining).
Figure 5 illustrates a comparison between immersion decellularization (top row) and perfusion decellularization (bottom row) of a rat heart.
Figure 6 shows a comparisons between immersion decellularization (top row) versus perfusion decellularization (bottom row) using rat kidney.
Figure 7 shows SEM photographs of decellularized kidney
Figure 8A shows a SEM photograph of a perfusiondecellularized heart, while Figure 8B shows a SEM photograph of an immersion decellularized heart.

### Detailed Description of the Invention

The present disclosure achieves engineering of cells and ECMs that, through physical as well as molecular interactions, direct control of cell behavior by controlling the environment of those cells. In particular, the present invention provides a method which results in engineered organs, tissues or bioreactors having perfusion decellularized ECM implanted with a population of cells, including combinations of cells, and subjected to culture conditions, e.g., including perfusion of soluble mediators, which ECM structure and culture conditions result in functional cells and capillary vessel lumen diameter that is substantially the same as in a corresponding native organ, e.g., about 5 µm to about 10 µm, or about 3 µm to about 20 µm. In particular, the method of the invention may provide for improved regulation of cell differentiation, growth, and phenotypic expression of stem cells, in particular adult stem cells, and partially differentiated progenitor cells, and improved maintenance of differentiated cell types, as a result of maintaining capillary vessel lumen diameter. It also includes the growth and functional maintenance of primary cells including fetal derived cells, e.g., organ-specific cells obtained from fetal cells or neonate cells (for instance, cells that are committed to a specific lineage but are not terminally differentiated).

Disclosed is the use of perfusion decellularized organ- or tissue-derived extracellular matrix (ECM) and systems useful to support recellularization of those matrices with vascular (e.g., endothelial) cells, or differentiation and/or maturation of stem or progenitor cells into endothelial cells, or maintenance or differentiated of primary cells, or any combination thereof, in the extracellular matrix vasculature of those matrices. Primary cells are cells obtained from an organism that generally are then cultured *in vitro,* although those cells do not proliferate indefinitely. Differentiated cells include primary cells and cells that have been differentiated *in vitro,* e.g., stem cells or progenitor cells in a perfusion decellularized matrix of the invention. In one embodiment, at least 5%, 10% or 20%, or more, of the differentiated cells have a functionally mature phenotype. A tissue is a group of cells with a common structure and function, e.g., epithelial tissue, connective tissue, muscle tissue (skeletal, cardiac, or smooth muscle), and nervous tissue, and includes a pliable sheet that covers or lines or connects organs. An organ is a collection of tissues (two or more) joined in structural unit to serve a common function. Organs include but are not limited to the brain, liver, pancreas, bone, heart, stomach, kidney, lungs, whole muscles, thymus, anus, and intestine. As used herein, an organ includes perfusable whole organs, or parts of an organ, or vascularized structures thereof, and a tissue includes any structures that contain vascularized tissues, e.g., a trachea.

Disclosed is the use of an organ- or tissue-specific extracellular matrix (ECM) scaffold for re-endothelialization using cells that may require differentiation or maturation, such as stem or progenitor cells. Differentiation is a process by which cells acquire a new phenotype that is distinct from the original cell population, e.g., distinct cellular gene and/or protein expression and/or function(s). Maturation further clarifies the phenotype of the cell population as having the normal mature functional capacity of a cell in an *in vivo* cell population. In one aspect of the disclosure, the scaffold is a perfusion decellularized ECM portion of an organ. In another aspect of the disclosure, the scaffold is a perfusion decellularized ECM organ.

Perfusion decellularized ECM from organs or tissues retains more of the native microstructure, including an intact vascular and/or microvascular system, compared to other decellularization techniques such as immersion based decellularization. For example, perfusion decellularized ECM from organs or tissues preserves the collagen content and other binding and signaling factors and vasculature structure, thus providing for a niche environment with native cues for functional differentiation or maintenance of cellular function of introduced cells. Disclosed is that perfusion decellularized ECM from organs or tissues is perfused with cells and/or media using the vasculature of the perfusion decellularized ECM under appropriate conditions, including appropriate pressure and flow to mimic the conditions normally found in the organism. The normal pressures of human sized organs is between about 40 to about 200 mm Hg with the resulting flow rate dependent upon the incoming perfusion vessel diameter. For a normal human heart the resulting perfusion flow is about 20 to about 200 mL/min/100 g. Using such a system, the seeded cells can achieve a greater seeding concentration of about 5x up to about 1000x greater than achieved under 2D cell culture conditions and, unlike a 2D culture system, the ECM environment allows for the further functional differentiation of cells, e.g., differentiation of progenitor cells into cells that demonstrate organ- or tissue-specific phenotypes having sustained function. In one aspect of the disclosure, the combination of culture conditions and source of ECM allows for the functional differentiation of cells introduced to the ECM.

Disclosed is that the method includes selecting a perfusion decellularized matrix of an organ or tissue and a population of cells including endothelial cells or progenitor cells capable of differentiation to endothelial cells. The selected perfusion decellularized matrix is contacted with the population of cells under conditions and for a period of time that provide for recellularization of the perfusion decellularized matrix and for progenitor cells, differentiation of cells in the population into functional cells. In one embodiment, the nanoparticles and microparticles are introduced and circulated with the cells through the vasculature. In one embodiment, the nanoparticles and microparticles are introduced and circulated through the vasculature after re-endothelialization. In one embodiment, the organ is a heart. In another embodiment, the organ is a liver. In another embodiment, the organ is a pancreas. In another embodiment, the organ is a lung.

In one embodiment, the invention provides a method to maintain capillary vessel lumen diameter in a re-endothelialized perfusion decellularized matrix. The method includes selecting a perfusion decellularized matrix of an organ or tissue and a population of stem cells capable of differentiation to endothelial cells or endothelial cells. The selected perfusion decellularized matrix is contacted with the cells under conditions and for a period of time that provide for re-endothelialization of the perfusion decellularized matrix and the endothelial cells or differentiation of the stem cells in the population into functional endothelial cells. In one embodiment, the stem cells are induced pluipotent stem (iPS) cells. In one embodiment, the stem cells are adult stem cells.

In one embodiment, a portion of an organ or tissue ECM is employed in the methods of the invention, e.g., an atrium or ventricle of a heart or interior structure of a pancreas including islets. In one embodiment, the portion is about 5 to about 10 mm in thickness. In one embodiment, the portion is about 70 to about 100 mm in thickness.

The ECM organ or tissue matrices may be obtained from any source including, without limitation, heart, liver, lungs, skeletal muscles, brain, pancreas, spleen, kidneys, uterus, eye, spinal cord, whole muscle, or bladder, or any portion thereof (e.g., an aortic valve, a mitral valve, a pulmonary valve, a tricuspid valve, a pulmonary vein, a pulmonary artery, coronary vasculature, septum, a right atrium, a left atrium, a right ventricle, or a left ventricle). A solid organ refers to an organ that has a "substantially closed" vasculature system. A "substantially closed" vasculature system with respect to an organ means that, upon perfusion with a liquid, the majority of the liquid is contained within the solid organ or pass out the native vascular structures and does not leak out of the solid organ, assuming the major vessels are cannulated, ligated, or otherwise restricted. Despite having a "substantially closed" vasculature system, many of the organs listed above have defined "entrance" and "exit" vessels which are useful for introducing and moving the liquid throughout the organ during perfusion. In addition, other types of vascularized organs or tissues such as, for example, all or portions of joints (e.g., knees, shoulders, or hips), anus, trachea, or spinal cord, can be perfusion decellularized. Further, avascular tissues such as, for example, cartilage or cornea, may be decellularized when part of a larger vascularized structures such as a whole leg.

Perfusion decellularized matrices of organs with a substantially closed vascular system are particularly useful because perfusion decellularization preserves the intact matrix and microenvironment, including an intact vascular and microvascular system, that vascular system may be utilized to deliver cells as well as nutrients and/or differentiation or maintenance factors, to the cells *in vitro.* Cells and nutrients and/or other factors may be delivered by other means, e.g., injection, or passive means, or a combination thereof. In one embodiment, a cell population of interest is perfused into the perfusion decellularized organ ECM allowing for the seeding into the interstitial space or matrix outside of the vascular conduits. This includes the active migration and/or homing of cells to their native microstructure, e.g. the homing of endothelial cells to the vasculature. In one embodiment, a cell population of interest is perfused into the perfusion decellularized ECM followed by a second cell population, e.g., a beta cell population is introduced followed by an endothelial cell population, where the endothelial cells remain in the vascular conduits as in their native microenvironment. In one embodiment, a cell population of interest is perfused into the perfusion decellularized ECM followed by a second cell population, e.g., an endothelial cell population is introduced followed by a population of cells that include beta cells, where the endothelial cells remain in the vascular conduits as in their native microenvironment. In another embodiment, two or more cell populations are combined and perfused together. In another embodiment, two or more distinct cell populations are introduced serially through either perfusion, direct injection or a combination of both. The particles of the invention are introduced to the re-endothelialized vasculature *ex vivo* to maintain, reduced a decrease in or enhance capillary vessel lumen diameter.

The cells may be introduced in media that support the proliferation, metabolism, and/or differentiation of the cells. Alternatively, after the cells have populated the ECM, the medium is changed to one that supports the proliferation, metabolism and differentiation of the cells. The cultured cells may exist in the ECM at physiological cell densities and, in the presence of media that support the proliferation, metabolism, and/or differentiation of the cells and/or the appropriate microenvironment in the ECM, allow for the maintenance and/or functional differentiation of the cells.

The cells and ECM may be xenogeneic or allogeneic. In one embodiment, partially or completely differentiated human cells and a perfusion decellularized organ or tissue from a small animal, e.g., a nonhuman mammal, can be combined. In one example, a perfusion decellularized liver matrix from a human is seeded with endothelial cells and partially differentiated human ES derived hepatocyte cells providing allogeneic or xenogeneic, respectively, cell seeded matrices.

### Perfusion Decellularized ECM

Studies have shown that connective tissue cells behave very differently in 3D as opposed to 2D cultures (Cukierman et al., Science, 294:1708 (2001)). For example, culture of fibroblasts on flat substrates induces a polarity that does not occur *in vivo.* Further, when fibroblasts and other cell types are cultured in 3D tissue-derived matrices, they develop mature integrin-containing focal adhesion complexes within minutes that resemble the complexes found *in vivo,* whereas only primitive adhesion complexes develop in 2D cultures or even simple 3D type I collagen gels or Matrigel. These adhesion complexes are required for appropriate growth factor-activated receptor signaling and rapid (within 5 minutes) initiation of synthesis of their own ECM components and factors that alter the ECM (Cukierman et al., 2001; Abbott, Nature, 424:870 (2003)). In addition, cells in ECM culture deposit autocrine growth factors into tissue-derived matrices, a process that may be required for appropriate presentation of the growth factor to target cells. Such factors are mainly secreted into the culture medium in 2D cultures.

As mentioned above, physical interactions with the ECM, in addition to chemical, molecular (e.g., soluble mediators), or genetic (cell-type) factors, may regulate cell fate. For example, ECM-based control of the cell may occur through multiple physical mechanisms, such as ECM geometry at the micro- and nanoscale, ECM elasticity, or mechanical signals transmitted from the ECM to the cells.

Disclosed is the use of engineered perfusion decellularized ECMs that allow for better control of cell behavior, e.g., from adult stem cells, through physical as well as molecular interactions. The perfusion decellularized matrices mimic the intricate and highly ordered nature of native ECM and the likely reciprocal interaction between cells and the ECM. In particular, the ECM may provide tissue-specific cues to stem or progenitor cells. In particular, distinct matrix proteins may be important for the specificity of ECM via their contribution to the architecture of the ECM or via their ability to interact with growth factors and/or the resident cells themselves.

Perfusion decellularization of tissue or organ ECM provides an intact ECM that has the ability to provide the structural, biochemical, and mechanical properties to enable functional cell differentiation and maintenance. Thus, perfusion decellularization of organs allows organs to serve as a tissue/organ specific bioreactor for stem or progenitor cell differentiation. Moreover, perfusion decellularization of organ or tissue ECM is superior to immersion in terms of preserving an intact matrix with structural and biochemical cues, including intact vasculature. In addition, perfusion decellularization provides advantages relative to immersion decellularization when tissue or organ thickness exceeds about 2 mm in thickness.

### Decellularization of Organs or Tissues

Decellularization generally includes the following steps: stabilization of the solid organ, e.g., a vascularized structure thereof, or tissue, decellularization of the solid organ or tissue, renaturation and/or neutralization of the solid organ or tissue, washing the solid organ, degradation of any DNA remaining on the organ, disinfection of the organ or tissue and homeostasis of the organ.

The initial step in decellularizing an organ vascularized structure or tissue is to cannulate the organ or tissue. The vessels, ducts, and/or cavities of an organ or tissue may be cannulated using methods and materials known in the art. Next, the cannulated organ vascuarlized structure or tissue is perfused with a cellular disruption medium. Perfusion through an organ can be multi-directional (e.g., antegrade and retrograde).

Langendorff perfusion of a heart is routine in the art, as is physiological perfusion (also known as four chamber working mode perfusion). See, for example, Dehnert, The Isolated Perfused Warm-Blooded Heart According to Langendorff, In Methods in Experimental Physiology and Pharmacology: Biological Measurement Techniques V. Biomesstechnik-Verlag March GmbH, West Germany, 1988.

Briefly, for Langendorff perfusion, the aorta is cannulated and attached to a reservoir containing physiological solution to allow the heart to function outside of the body for a specified duration of time. To achieve perfusion decellularization the protocol has been modified to perfuse a cellular disruption medium delivered in a retrograde direction down the aorta either at a constant flow rate delivered, for example, by an infusion or roller pump or by a constant hydrostatic pressure pump. In both instances, the aortic valves are forced shut and the perfusion fluid is directed into the coronary ostia (thereby perfusing, via antegrade, the entire ventricular mass of the heart), which then drains into the right atrium via the coronary sinus. For working mode perfusion, a second cannula is connected to the left atrium and perfusion can be changed to retrograde.

In one embodiment, a physiological solution includes phosphate buffer saline (PBS). In one embodiment, the physiological solution is a physiologically compatible buffer supplemented with, e.g., nutritional supplements (for instance, glucose). For example, for heart, the physiological solution may be Modified Krebs-Henseleit buffer having 118 mM NaCl, 4.7 mM KCl, 1.2 mM MgSO₄, 1.2 mM KH₂PO₄, 25 mM NaHCO₃, 11 mM glucose, 1.75 mM CaCl₂, 2.0 mM pyruvate and 5 U/L insulin; or Krebs buffer containing 118 mM NaCl, 4.7 mM KCl, 25 mM NaHCO₃, 1.2 mM MgSO₄, 1.2 mM KH₂PO₄, 2 mM CaCl₂ gassed with 95% O₂, 5% CO₂. Hearts may be perfused with glucose (e.g., about 11 mM) as a sole substrate or in combination with about 1 or 1.2 mM palmitate. For kidney, the physiological solution may be KPS-1® Kidney Perfusion Solution. For liver, the physiological solution may be

Krebs-Henseleit buffer having 118 mM NaCl, 4.7 mM KCl, 1.2 mM MgSO₄, 1.2 mM KH₂PO₄, 26 mM NaHCO₃, 8 mM glucose, and 1.25 mM CaCl₂ supplemented with 2% BSA.

Methods are known in the art for perfusing other organ or tissues. By way of example, the following references describe the perfusion of lung, liver, kidney, brain, and limbs. Van Putte et al., Ann. Thorac. Surg., 74(3):893 (2002); den Butter et al., Transpl. Int., 8:466 (1995); Firth et al., Clin. Sci. (Lond.), 77(6):657 (1989); Mazzetti et al., Brain Res., 999(1):81 (2004); Wagner et al., J. Artif. Organs, 6(3):183 (2003).

One or more cellular disruption media may be used to decellularize an organ or tissue. A cellular disruption medium generally includes at least one detergent such as but not limited to SDS, PEG, CHAPS or Triton X. A cellular disruption medium can include water such that the medium is osmotically incompatible with the cells. Alternatively, a cellular disruption medium can include a buffer (e.g., PBS) for osmotic compatibility with the cells. Cellular disruption media also may include enzymes such as, without limitation, one or more collagenases, one or more dispases, one or more DNases, or a protease such as trypsin. In some instances, cellular disruption media also or alternatively may include inhibitors of one or more enzymes (e.g., protease inhibitors, nuclease inhibitors, and/or collegenase inhibitors).

Disclosed is further that a cannulated organ or tissue may be perfused sequentially with two different cellular disruption media. For example, the first cellular disruption medium may include an anionic detergent such as SDS and the second cellular disruption medium can include an ionic detergent such as Triton X. Following perfusion with at least one cellular disruption medium, a cannulated organ or tissue may be perfused, for example, with wash solutions and/or solutions containing one or more enzymes such as those disclosed herein.

Alternating the direction of perfusion (e.g., antegrade and retrograde) may assist in decellularizing the entire organ or tissue. Decellularization generally decellularizes the organ from the inside out, resulting in very little damage to the ECM. An organ or tissue may be decellularized at a suitable temperature between 4 and 40°C. Depending upon the size and weight of an organ or tissue and the particular detergent(s) and concentration of detergent(s) in the cellular disruption medium, an organ or tissue generally is perfused from about 0.05 hours to about 5 hours, per gram of solid organ or tissue (generally > 50 grams), or about 2 hours to about 12 hours, per gram of solid organ or tissue for organs (generally < 50 grams), with cellular disruption medium. Including washes, an organ may be perfused for up to about 0.75 hours to about 10 hours per gram of solid organ or tissue (generally >50 grams), or about 12 hours to about 72 hours, per gram of tissue (generally <50 grams). Decellularization time is dependent upon the vascular and cellular density of the organ or tissue with limited scaling for overall mass. Therefore, as general guidance the time ranges and masses above are provided. Perfusion generally is adjusted to physiologic conditions including pulsatile flow, rate and pressure.

A decellularized organ or tissue has the extracellular matrix (ECM) component of all or most regions of the organ or tissue, including ECM components of the vascular tree. ECM components can include any or all of the following: fibronectin, fibrillin, laminin, elastin, members of the collagen family (e.g., collagen I, III, and IV), ECM associated growth proteins including growth factors and cytokines, glycosaminoglycans, ground substance, reticular fibers and thrombospondin, which can remain organized as defined structures such as the basal lamina. Successful decellularization is defined as the absence of detectable myofilaments, endothelial cells, smooth muscle cells, and nuclei in histologic sections using standard histological staining procedures or removal of over 97% of detectable DNA as measured by fluorometric assay. Residual cell debris may be removed from the decellularized organ or tissue.

The morphology and the architecture of the ECM is maintained during and following the process of decellularization. "Morphology" as used herein refers to the overall shape of the organ, tissue or of the ECM, while "architecture" as used herein refers to the exterior surface, the interior surface, and the ECM therebetween.

The morphology and architecture of the ECM may be examined visually and/or histologically. For example, the basal lamina on the exterior surface of a solid organ or within the vasculature of an organ or tissue should not be removed or significantly damaged due to perfusion decellularization. In addition, the fibrils of the ECM should be similar to or significantly unchanged from that of an organ or tissue that has not been decellularized.

One or more compounds may be applied in or on a decellularized organ or tissue to, for example, preserve the decellularized organ, or to prepare the decellularized organ or tissue for recellularization and/or to assist or stimulate cells during the recellularization process. Such compounds include, but are not limited to, one or more growth factors (e.g., VEGF, DKK-1, FGF, BMP-1, BMP-4, SDF-1, IGF, and HGF), immune modulating agents (e.g., cytokines, glucocorticoids, IL2R antagonist, leucotriene antagonists), and/or factors that modify the coagulation cascade (e.g., aspirin, heparin-binding proteins, and heparin). In addition, a decellularized organ or tissue may be further treated with, for example, irradiation (e.g., UV, gamma) to reduce or eliminate the presence of any type of microorganism remaining on or in a decellularized organ or tissue.

### Exemplary Perfusion Decellularization of Heart

### PEG Decellularization Protocol

Hearts are washed in 200 ml PBS containing 100 U/mL penicillin, 0.1 mg/mL Streptomycin, 0.25 µg/mL Amphotericin B, 1000 U of hepatin, and 2 mg of Adenocard with no recirculation. Hearts are then decellularized with 35 ml polyethyleneglycol (PEG; 1 g/mL) for up to 30 minutes with manual recirculation. The organ is then washed with 500 mL PBS for up to 24 hours using a pump for recirculation. The washing step is repeated at least twice for at least 24 hours each time. Hearts are exposed to 35 ml DNase I (70 U/mL) for at least 1 hour with manual recirculation. The organs are washed again with 500 ml PBS for at least 24 hours.

### Triton X and Trypsin Decellularization Protocol

Hearts are washed in 200 ml PBS containing 100 U/mL Penicillin, 0.1 mg/mL Streptomycin, 0.25 µg/mL Amphotericin B, 1000 U of hepatin, and 2 mg of Adenocard for at least about 20 minutes with no recirculation. Hearts are then decellularized with 0.05% Trypsin for 30 minutes followed by perfusion with 500 mL PBS containing 5% Triton-X and 0.1% ammonium-hydroxide for about 6 hours. Hearts are perfused with deionized water for about 1 hour, and then perfused with PBS for 12 hours. Hearts are then washed 3 times for 24 hours each time in 500 mL PBS using a pump for recirculation. The hearts are perfused with 35 ml DNase I (70 U/mL) for 1 hour with manual recirculation and washed twice in 500 mL PBS for at least about 24 hours each time using a pump for recirculation.

### 1% SDS Decellularization Protocol

Hearts are washed in 200 mL PBS containing 100 U/mL Penicillin, 0.1 mg/mL Streptomycin, 0.25 µg/mL Amphotericin B, 1000 U of hepatin, and 2 mg of Adenocard for at least about 20 minutes with no recirculation. The hearts are decellularized with 500 mL water containing 1% SDS for at least about 6 hours using a pump for recirculation. The hearts are then washed with deionized water for about 1 hour and washed with PBS for about 12 hours. The hearts are washed three times with 500 mL PBS for at least about 24 hours each time using a pump for recirculation. The heart is then perfused with 35 ml DNase I (70 U/mL) for about 1 hour using manual recirculation, and washed three times with 500 mL PBS for at least about 24 hours each time using a pump for recirculation.

### Triton X Decellularization Protocol

Hearts are washed with 200 mL PBS containing 100 U/mL Penicillin, 0.1 mg/ml Streptomycin, 0.25 µg/mL Amphotericin B, 1000 U of hepatin, and 2 mg of Adenocard (adenosine) for at least about 20 minutes with no recirculation. Hearts are then decellularized with 500 mL water containing 5% Triton X and 0.1% ammonium hydroxide for at least 6 hours using a pump for recirculation. Hearts are then perfused with deionized water for about 1 hour and then with PBS for about 12 hours. Hearts are washed by perfusing with 500 mL PBS 3 times for at least 24 hours each time using a pump for recirculation. Hearts are then perfused with 35 ml DNase I (70 U/mL) for about 1 hour using manual recirculation, and washed three times in 500 ml PBS for about 24 hours each time.

Hearts may be perfused at a coronary perfusion pressure of 60 cm H₂O. Although not required, the hearts may be mounted in a decellularization chamber and completely submerged and perfused with PBS containing antibiotics for 72 hours in recirculation mode at a continuous flow of 5 mL/minute to wash out as many cellular components and detergent as possible.

### Detection of Cardiac Decellularization

Successful decellularization may be measured by the lack of myofilaments and nuclei in histologic sections. Successful preservation of vascular structures may be assessed by perfusion with 2% Evans Blue prior to embedding tissue sections. Highly efficient decellularization is observed when a heart is first perfused antegradely with an ionic detergent (1% sodium-dodecylsulfate (SDS), approximately 0.03 M) dissolved in deionized H₂O at a constant coronary perfusion pressure and then perfused antegradely with a non-ionic detergent (1% Triton X-100) to remove the SDS and presumably to renature the extracellular matrix (ECM) proteins. Intermittently, the heart may be perfused retrogradely with phosphate buffered solution to clear obstructed capillaries and small vessels.

To demonstrate intact vascular structures following decellularization, a decellularized heart may be stained via Langendorff perfusion with Evans Blue to stain vascular basement membrane and quantify macro- and micro-vascular density. Further, polystyrene particles may be perfused into and through a heart to quantify coronary volume, the level of vessel leakage, and to assess the distribution of perfusion by analyzing coronary effluent and tissue sections. A combination of three criteria are assessed and compared to isolated non-decellularized heart: 1) an even distribution of polystyrene particles, 2) significant change in leakiness at some level 3) microvascular density.

Fiber orientation may be assessed by the polarized-light microscopy technique of Tower et al. (Ann Biomed Eng., 30(10):1221 (2002), which can be applied in real-time to a sample subjected to uniaxial or biaxial stress. During Langendorff perfusion, basic mechanical properties of the decellularised ECM are recorded (compliance, elasticity, burst pressure) and compared to freshly isolated hearts.

### Exemplary Perfusion Decellularization of Liver

For liver isolation, the caval vein is exposed through a median laparotomy, dissected and canulated using a mouse aortic canula (Radnoti Glass, Monrovia, Calif.). The hepatic artery and vein and the bile duct are transsected and the liver was carefully removed from the abdomen and submerged in sterile PBS (Hyclone, Logan, Utah) to minimize pulling force on portal vein. 15 minutes of heparinized PBS perfusion is followed by 2-12 hours of perfusion with 1% SDS (Invitrogen, Carlsbad, Calif.) in deionized water and 15 minutes of 1% Triton-X (Sigma, St. Louis, Mo.) in deionized water. The liver is then continuously perfused with antibiotic containing PBS (100 U/ml penicillin-G (Gibco, Carlsbad, Calif.), 100 U/ml streptomycin (Gibco, Carlsbad, Calif.), 0.25 µg/ml Amphotericin B (Sigma, St. Louis, Mo.)) for 124 hours.

120 minutes of SDS perfusion followed by perfusion with Triton-X 100 are sufficient to generate a completely decellularized liver. Movat pentachrome staining of decellularized liver confirms retention of characteristic hepatic organization with central vein and portal space containing hepatic artery, bile duct and portal vein.

### Recellularization of Organs or Tissues

A decellularized organ or tissue is contacted with a population of cells, either differentiated (mature or primary) cells, stem cells, or partially differentiated cells including different types of cells. Thus, the cells can be totipotent cells, pluripotent cells, or multipotent cells, and can be uncommitted or committed, and may be single-lineage cells. The cells may be undifferentiated cells, partially differentiated cells, or fully differentiated cells including fetal derived cells. Cells may include progenitor cells, precursor cells, or "adult" derived stem cells including umbilical cord cells and fetal stem cells. Cells useful in the matrices of the invention include iPS cells.

Examples of cells that can be used to recellularize an organ or tissue include, without limitation, stem cells, umbilical cord blood cells, tissue-derived stem or progenitor cells, bone marrow-derived step or progenitor cells, blood-derived stem or progenitor cells, mesenchymal stem cells (MSC), skeletal muscle-derived cells, multipotent adult progentitor cells (MAPC), or iPS cells Additional cells that can be used include cardiac stem cells (CSC), multipotent adult cardiac-derived stem cells, cardiac fibroblasts, cardiac microvasculature endothelial cells, aortic endothelial cells, coronary endothelial cells, microvascular endothelial cells, venous endothelial cells, arterial endothelial cells, smooth muscle cells, cardiomyocytes, hepatocytes, beta-cells, keratinocytes, purkinji fibers, neurons, bile duct epithelial call, islet cells, pneumocytes, clara cells, brush boarder cells, or podocytes. Bone marrow-derived stem cells such as bone marrow mononuclear cells (BM-MNC), endothelial or vascular stem or progenitor cells, and peripheral blood-derived stem cells such as endothelial progenitor cells (EPC) may also be used as cells.

The number of cells that are introduced into and onto a perfusion decellularized scaffold may depend both the organ (e.g., which organ, the size and weight of the organ) or tissue and the type and developmental stage of the regenerative cells. Different types of cells may have different tendencies as to the population density those cells will reach. Similarly, different organ or tissues may be cellularized at different densities. By way of example, a decellularized organ or tissue can be "seeded" with at least about 1,000 (e.g., at least 10,000, 100,000, 1,000,000, 10,000,000, or 100,000,000) cells; or can have from about 1,000 cells/mg tissue (wet weight, e.g., prior to decellularization) to about 10,000,000 cells/mg tissue (wet weight) attached thereto.

Cells can be introduced ("seeded") into a decellularized organ or tissue by injection into one or more locations. In addition, more than one type of cell may be introduced into a decellularized organ or tissue. For example, a population of differentiated cell types can be injected at multiple positions in a decellularized organ or tissue or different cell types may be injected into different portions of a decellularized organ or tissue. Alternatively, or in addition to injection, cells or a cocktail of cells may be introduced by perfusion into a cannulated decellularized organ or tissue. For example, cells can be perfused into a decellularized organ using a perfusion medium, which can then be changed to an expansion and/or differentiation medium to induce growth and/or differentiation of the cells. Location specific differentiation may be achieved by placing cells into the various locations within the organ, e.g., into regions of the heart, such as, atrial, ventricular or nodal.

During recellularization, an organ or tissue is maintained under conditions in which at least some of the cells can multiply and/or differentiate within and on the decellularized organ or tissue. Those conditions include, without limitation, the appropriate temperature and/or pressure, electrical and/or mechanical activity, force, the appropriate amounts of O₂ and/or CO₂, an appropriate amount of humidity, and sterile or near-sterile conditions. During recellularization, the decellularized organ or tissue and the regenerative cells attached thereto are maintained in a suitable environment. For example, the cells may require a nutritional supplement (e.g., nutrients and/or a carbon source such as glucose), exogenous hormones or growth factors, and/or a particular pH.

Cells may be allogeneic to a decellularized organ or tissue (e.g., a human decellularized organ or tissue seeded with human cells), or cells may be xenogeneic to a decellularized organ or tissue (e.g., a pig decellularized organ or tissue seeded with human cells). "Allogeneic" as used herein refers to cells obtained from the same species as that from which the organ or tissue originated (e.g., related or unrelated individuals), while "xenogeneic" as used herein refers to cells obtained from a species different than that from which the organ or tissue originated.

Stem or progenitor media may contain a variety of components including, for example, KODMEM medium (Knockout Dulbecco's Modified Eagle's Medium), DMEM, Ham's F12 medium, FBS (fetal bovine serum), FGF2 (fibroblast growth factor 2), KSR or hLIF (human leukemia inhibitory factor). The cell differentiation media may also contain supplements such as L-Glutamine, NEAA (non-essential amino acids), P/S (penicillin/streptomycin), N2, B27 and beta-mercaptoethanol. It is contemplated that additional factors may be added to the cell differentiationmedia, including, but not limited to, fibronectin, laminin, heparin, heparin sulfate, retinoic acid, members of the epidermal growth factor family (EGFs), members of the fibroblast growth factor family (FGFs) including FGF2, FGF7, FGF8, and/or FGF10, members of the platelet derived growth factor family (PDGFs), transforming growth factor (TGF)/bone morphogenetic protein (BMP)/growth and differentiation factor (GDF) factor family antagonists including but not limited to noggin, follistatin, chordin, gremlin, cerberus/DAN family proteins, ventropin, high dose activin, and amnionless or variants or functional fragments thereof. TGF/BMP/GDF antagonists could also be added in the form of TGF/BMP/GDF receptor-Fc chimeras. Other factors that may be added include molecules that can activate or inactivate signaling through Notch receptor family, including but not limited to proteins of the Delta-like and Jagged families as well as inhibitors of Notch processing or cleavage, or variants or functional fragments thereof. Other growth factors may include members of the insulin like growth factor family (IGF), insulin, the wingless related (WNT) factor family, and the hedgehog factor family or variants or functional fragments thereof. Additional factors may be added to promote mesendoderm stem/progenitor, endoderm stem/progenitor, mesoderm stem/progenitor, or definitive endoderm stem/progenitor proliferation and survival as well as survival and differentiation of derivatives of these progenitors.

In one embodiment, perfusion decellularized matrices are combined with iPS differentiated using the embryoid body (EB) method. For example, human iPS cell lines reprogrammed by transduction, e.g., lentiviral-mediated transduction, of transcription factors (*OCT4, SOX2, NANOG* and *LIN28;* Oct3/4, Sox2, Klf4, and c-Myc; or Oct3/4, Sox2, and Klf4) are employed. iPS clones of fetal origin or of newborn origin may be employed. iPS cells may be maintained on irradiated mouse embryonic fibroblasts (MEFs) at a density of 19,500 cells/cm² in 6-well culture plates (Nunc) in DMEM/F12 culture medium supplemented with 20% KnockOut serum replacer (Invitrogen), 0.1 mmol/L nonessential amino acids, 1 mmol/L L-glutamine, and 0.1 mmol/L β-mercaptoethanol (Sigma). In addition, the medium may be supplemented with 100 ng/mL, zebrafish basic fibroblast growth factor for iPS cells. iPS lines may also be maintained on gelatinized 100-mm dishes in DMEM (Sigma-Aldrich) containing 15% fetal calf serum (FCS; Sigma-Aldrich), 0.1 µmol/L 2-mercaptoethanol (2ME), and 1,000 units/ml LIF (Chemicon International). For differentiation, these cells may treated with 0.25% Trypsin/ethylenediaminetetraacetic acid (GIBCO), and transferred to gelatinized 6-well plates in α-minimum essential medium (GIBCO) supplemented with 10% FCS and 0.05 µmol/L 2ME, at a concentration of 3 × 10⁴ cells/well.

Colonies may be detached from culture plates by incubating with 1 mg/mL dispase (Gibco) solution at 37°C for 8 to 15 minutes and placed in ultralow attachment plates in suspension culture, e.g., for 4 days. During suspension culture, the medium may be changed at day 1 followed by culture for another 3 days without medium change. EBs are then plated on 0.1% gelatin-coated culture plates, e.g., at the density or 50 to 100 EBs per well, or in the perfusion decellularized ECM and cultured in differentiation medium (e.g., changed daily).

In some instances, an organ or tissue generated by the methods described herein is to be transplanted into a patient. In those cases, the cells used to recellularize a decellularized organ or tissue can be obtained from the patient such that the cells are "autologous" to the patient. Cells from a patient can be obtained from, for example, blood, bone marrow, tissues, or organs at different stages of life (e.g., prenatally, neonatally or perinatally, during adolescence, or as an adult) using methods known in the art. Alternatively, cells used to recellularize a decellularized organ or tissue may be syngeneic (i.e., from an identical twin) to the patient, cells can be human lymphocyte antigen (HLA)-matched cells from, for example, a relative of the patient or an HLA-matched individual unrelated to the patient, or cells can be allogeneic to the patient from, for example, a non-HLA-matched donor.

Irrespective of the source of the cells (e.g., autologous or not), the decellularized solid organ can be autologous, allogeneic or xenogeneic to a patient.

The progress of cells can be monitored during recellularization. For example, the number of cells on or in an organ or tissue can be evaluated by taking a biopsy at one or more time points during recellularization. In addition, the amount of differentiation that cells have undergone can be monitored by determining whether or not various markers are present in a cell or a population of cells. Markers associated with different cells types and different stages of differentiation for those cell types are known in the art, and can be readily detected using antibodies and standard immunoassays. See, for example, Current Protocols in Immunology, 2005, Coligan et al., Eds., John Wiley & Sons, Chapters 3 and 11. Nucleic acid assays as well as morphological and/or histological evaluation can be used to monitor recellularization.

The recellularized graft is continuously perfused. Cell viability is maintained during culture, and quantification of TUNEL-positive cells may be conducted, e.g., to determine cells that are apoptotic.

### Controlled System for Decellularizing and/or Recellularizing An Organ or Tissue

A system (e.g., a bioreactor) for decellularizing and/or recellularizing an organ or tissue generally includes at least one cannulation device for cannulating an organ or tissue, a perfusion apparatus for perfusing the organ or tissue through the cannula(s), and means (e.g., a containment system) to maintain a sterile environment for the organ or tissue. Cannulation and perfusion are well-known techniques in the art. A cannulation device generally includes size-appropriate hollow tubing for introducing into a vessel, duct, and/or cavity of an organ or tissue. Typically, one or more vessels, ducts, and/or cavities are cannulated in an organ. A perfusion apparatus can include a holding container for the liquid (e.g., a cellular disruption medium) and a mechanism for moving the liquid through the organ (e.g., a pump, air pressure, gravity) via the one or more cannulae. The sterility of an organ or tissue during decellularization and/or recellularization can be maintained using a variety of techniques known in the art such as controlling and filtering the air flow and/or perfusing with, for example, antibiotics, anti-fungals or other anti-microbials to prevent the growth of unwanted microorganisms.

A system to decellularize and recellularize organ or tissues as described herein can possess the ability to monitor certain perfusion characteristics (e.g., pressure, volume, flow pattern, temperature, gases, pH), mechanical forces (e.g., ventricular wall motion and stress), and electrical stimulation (e.g., pacing). As the coronary vascular bed changes over the course of decellularization and recellularization (e.g., vascular resistance, volume), a pressure-regulated perfusion apparatus is advantageous to avoid large fluctuations. The effectiveness of perfusion can be evaluated in the effluent and in tissue sections. Perfusion volume, flow pattern, temperature, partial O₂ and CO₂ pressures and pH can be monitored using standard methods.

Sensors can be used to monitor the system (e.g., bioreactor) and/or the organ or tissue. Sonomicromentry, micromanometry, and/or conductance measurements can be used to acquire pressure-volume or preload recruitable stroke work information relative to myocardial wall motion and performance. For example, sensors can be used to monitor the pressure of a liquid moving through a cannulated organ or tissue; the ambient temperature in the system and/or the temperature of the organ or tissue; the pH and/or the rate of flow of a liquid moving through the cannulated organ or tissue; and/or the biological activity of a recellularizing organ or tissue. In addition to having sensors for monitoring such features, a system for decellularizing and/or recellularizing an organ or tissue also can include means for maintaining or adjusting such features. Means for maintaining or adjusting such features can include components such as a thermometer, a thermostat, electrodes, pressure sensors, overflow valves, valves for changing the rate of flow of a liquid, valves for opening and closing fluid connections to solutions used for changing the pH of a solution, a balloon, an external pacemaker, and/or a compliance chamber. To help ensure stable conditions (e.g., temperature), the chambers, reservoirs and tubings can be water-jacketed.

It can be advantageous during recellularization to place a mechanical load on the organ and the cells attached thereto. As an example, a balloon inserted into the left ventricle via the left atrium can be used to place mechanical stress on a heart. A piston pump that allows adjustment of volume and rate can be connected to the balloon to simulate left ventricular wall motion and stress. To monitor wall motion and stress, left ventricular wall motion and pressure can be measured using micromanometry and/or sonomicrometry. In some embodiments, an external pacemaker can be connected to a piston pump to provide synchronized stimulation with each deflation of the ventricular balloon (which is equivalent to the systole). Peripheral ECG can be recorded from the heart surface to allow for the adjustment of pacing voltage, the monitoring of de- and repolarization, and to provide a simplified surface map of the recellularizing or recellularized heart.

Mechanical ventricular distention can also be achieved by attaching a peristaltic pump to a canula inserted into the left ventricle through the left atrium. Similar to the procedure described above involving a balloon, ventricular distention achieved by periodic fluid movement (e.g., pulsatile flow) through the canula can be synchronized with electrical stimulation.

Using the methods and materials disclosed herein, a mammalian heart can be decellularized and recellularized and, when maintained under the appropriate conditions, a functional heart that undergoes contractile function and responds to pacing stimuli and/or pharmacologic agents can be generated.

A system for generating an organ or tissue may be controlled by a computer-readable storage medium in combination with a programmable processor (e.g., a computer-readable storage medium as used herein has instructions stored thereon for causing a programmable processor to perform particular steps). For example, such a storage medium, in combination with a programmable processor, may receive and process information from one or more of the sensors. Such a storage medium in conjunction with a programmable processor also can transmit information and instructions back to the bioreactor and/or the organ or tissue.

An organ or tissue undergoing recellularization may be monitored for biological activity. The biological activity can be that of the organ or tissue itself such as for cardiac tissue, electrical activity, mechanical activity, mechanical pressure, contractility, and/or wall stress of the organ or tissue. In addition, the biological activity of the cells attached to the organ or tissue may be monitored, for example, for ion transport/exchange activity, cell division, and/or cell viability. See, for example, Laboratory Textbook of Anatomy and Physiology (2001, Wood, Prentice Hall) and Current Protocols in Cell Biology (2001, Bonifacino et al., Eds, John Wiley & Sons). As discussed above, it may be useful to simulate an active load on an organ during recellularization. A computer-readable storage medium, in combination with a programmable processor, may be used to coordinate the components necessary to monitor and maintain an active load on an organ or tissue.

The weight of an organ or tissue may be entered into a computer-readable storage medium as described herein, which, in combination with a programmable processor, can calculate exposure times and perfusion pressures for that particular organ or tissue. Such a storage medium may record preload and afterload (the pressure before and after perfusion, respectively) and the rate of flow. For example, a computer-readable storage medium in combination with a programmable processor can adjust the perfusion pressure, the direction of perfusion, and/or the type of perfusion solution via one or more pumps and/or valve controls.

The invention will be further described in the following examples, which do not limit the scope of the invention defined in the claims.

### Example 1

### Comparison of Perfusion vs. Immersion

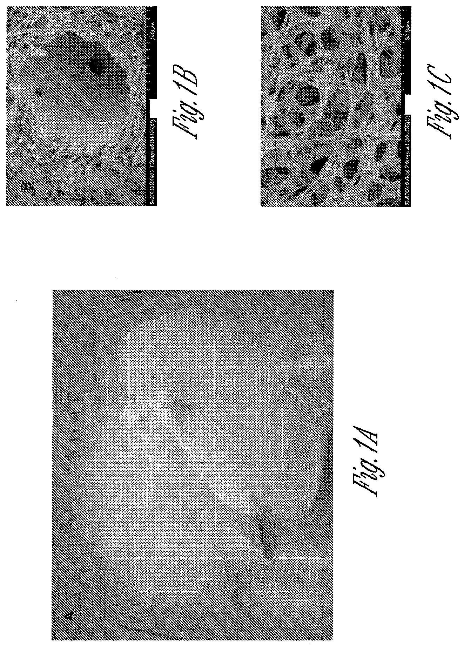

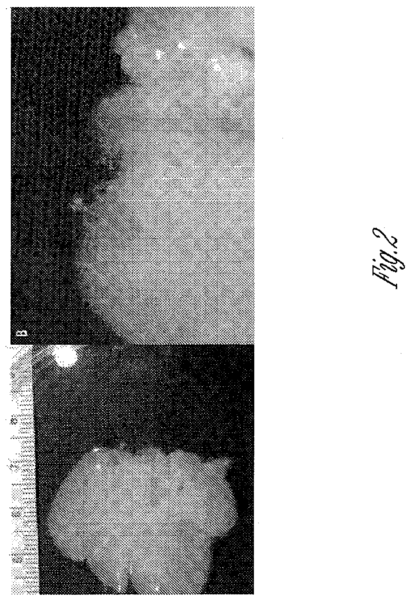

Figure 1A shows a photograph of a porcine liver that was perfusion decellularized, and Figure 1B and 1C show SEM of a vessel and the parenchymal matrix, respectively, of the perfusion decellularized porcine liver. These photographs show the vascular conduits and the matrix integrity of a perfusion decellularized organ. On the other hand, Figure 2 shows a gross view of an immersion decellularized rat liver, in which fraying of the matrix can be seen at both low (left) and high (right) magnification.

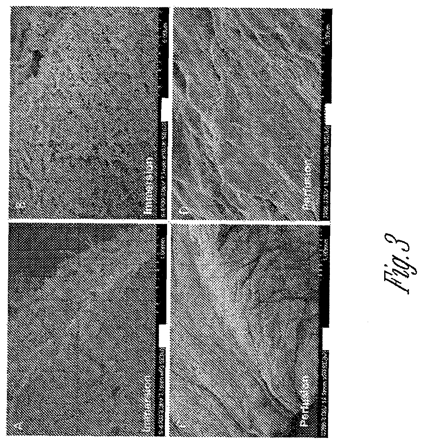

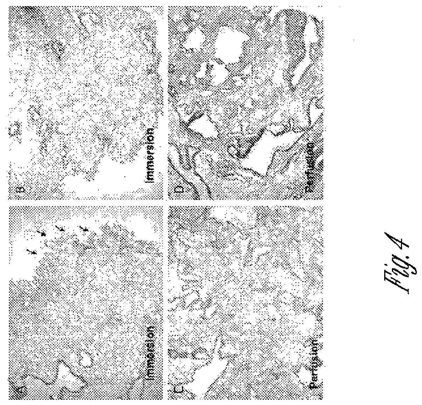

Figure 3 shows SEM of immersion decellularized rat liver (A and B) and perfusion decellularized rat liver (C and D). These results clearly indicate that immersion decellularization significantly compromised the organ capsule (Glisson's capsule), while perfusion decellularization retained the capsule. In addition, Figure 4 shows histology of immersion decellularized liver (A, H&E staining; B, Trichrome staining) and perfusion decellularized liver (C, H&E staining; D, Trichrome staining). The immersion decellularized rat liver did not retain cells or dye upon injection.

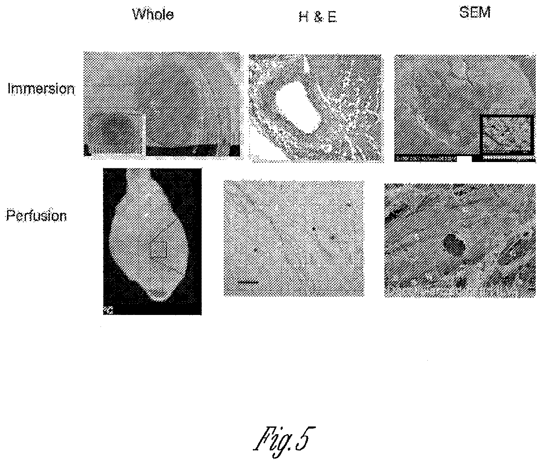

Figure 5 shows a comparison between immersion decellularization (top row) and perfusion decellularization (bottom row) of a rat heart. The photographs in the left column show the whole organ. As can be seen from the two photographs, the perfusion decellularized organ (bottom left) is much more translucent than the immersion decellularized organ (top left), which retains the iron-rich "brown-red" color of cadaveric muscle tissue and appears to still contain cells. The photographs in the middle column show the H&E staining pattern of the decellularized tissues. The staining shows that a number of cells, both within the parenchyma and in the walls of the vasculature, remain following immersion decellularization (top middle), while virtually every cell and also the cellular debris is removed following perfusion decellularization (bottom middle) even as patent vascular conduits are evident. In addition, the scanning electron micrographs in the right column show that there is a significant difference in the ultrastructure of the matrix following immersion (top right) vs. perfusion (bottom right) decellularization. Again, complete retention of cellular components throughout the cross section of the myocardium was observed in all the walls of the immersion-decellularized heart, but almost a complete loss of these cellular components was observed in the perfusion-decellularized heart along with the retention of spatial and architectural features of the intact myocardium including vascular conduits. For example, the perfusion-decellularized matrix retained the architectural features within the matrix including weaves (w), coils (c) and struts (s) despite the complete loss of cells.

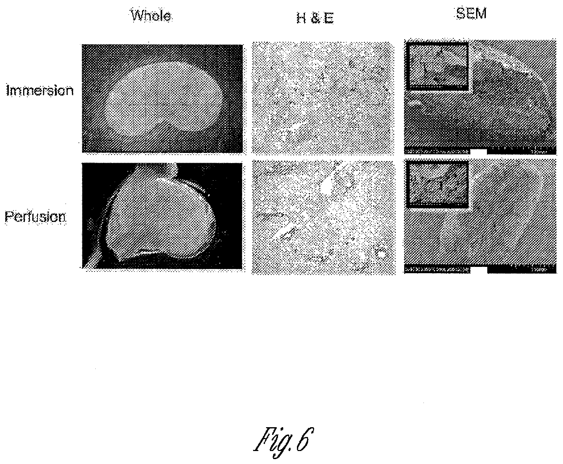

Figure 6 shows the same comparisons (immersion decellularization (top row) vs. perfusion decellularization (bottom row) using rat kidney. Unlike heart, the immersion-decellularized whole kidney (top left) looks grossly similar to the perfusion-decellularized whole kidney (bottom left) in that both are fairly translucent. However, in the perfusion-decellularized kidney, the network of vascular conduits within the perfusion-decellularized organ is more obvious and a greater degree of branching can be visualized than in the immersion-decellularied construct. Furthermore, the perfusion-decellularized kidney retains an intact organ capsule, is surrounded by mesentery, and, as shown, can be decellularized along with the attached adrenal gland. The photographs in the center column show the H&E staining pattern of the two tissues. The staining shows that cellular components and/or debris and possibly even intact nuclei (purple stain) remain following immersion-decelluarization (top center), while virtually every cell and/or all cellular debris is removed following perfusion-decellularization (bottom center). Likewise, the SEM photographs demonstrate that the immersion-decellularized kidney matrix (top right) suffered much more damage than did the perfusion-decellularized kidney matrix (bottom right). In the immersion-decellularized kidney, the organ capsule is missing or damaged such that surface "holes" or fraying of the matrix are obvious, whereas, in the perfusion decellularized organ, the capsule is intact.

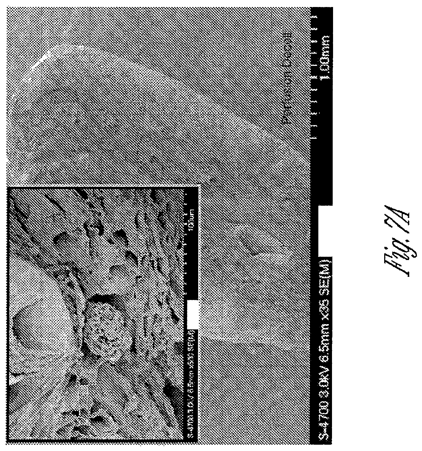

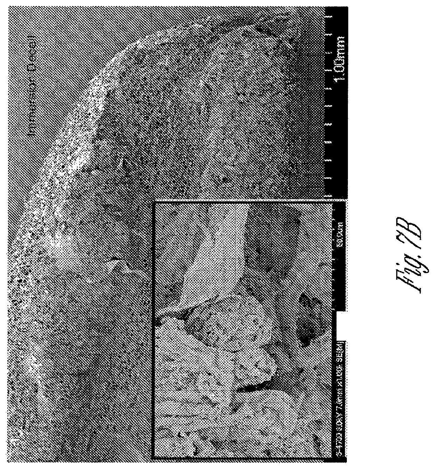

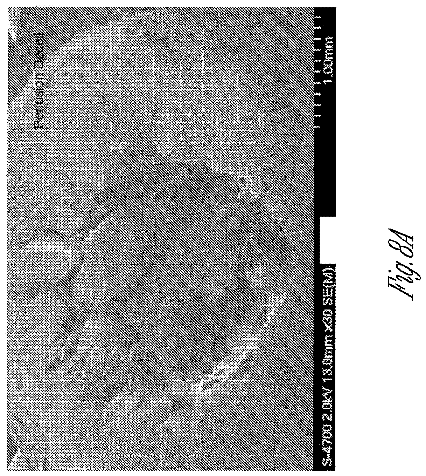

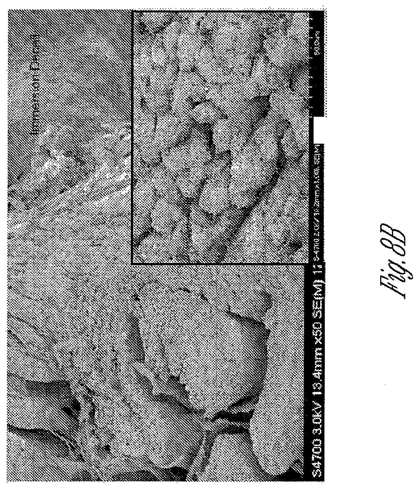

Figure 7 shows SEM photographs of decellularized kidney. Figure 7A shows a perfusion-decellularized kidney, while Figure 7B shows an immersion-decellularized kidney. Figure 8A shows a SEM photograph of a perfusion-decellularized heart, while Figure 8B shows a SEM photograph of an immersion-decellularized heart. These images further demonstrate the damage that immersion-decellularization caused to the ultrastructure of the organ, and the viability of the matrix following perfusion-decellularization.

### Example 2

### Exemplary Particles Useful in the Methods of the Invention

The particles useful in the methods of the invention include nanoparticles or microparticles , e.g., nanospheres or microspheres which may be formed of many different biocompatible materials, e.g., synthetic materials, biologic (natural) materials, or modified biologic materials, that may be degradable or non-degradable. Examples of materials from which the nanoparticles or microparticles may be formed include, but are not limited to, alignate, polysaccharide, collagen, dextran, hyaluronic acid, glass, ceramic, metal including titanium, particles with an iron core, PLA, PGA, PLA/PGA, monodisperse melamine resin particles, polystyrene, nylon, PMMA, and the like. Suitable polymeric materials may include, by way of example and not by way of limitation the following polymers: polyoxides, such as poly(ethylene oxide) and poly(propylene oxide); polyesters, such as poly(ethylene terepthalate); polyurethane; polysulfonate; polysiloxanes, such as poly(dimethyl siloxane); polysulfide; polyacetylene; polysulfone; polysulfonamide; polyamides such as polycaprolactam and poly(hexamethylene adipamide); polyimine; polyurea; heterocyclic polymers such as polyvinyl pyridine and polyvinyl pyrrolidinone; naturally occurring polymers such as natural rubber, gelatin, cellulose; polycarbonate; polyanhydride; and polyalkenes such as polyethylene, polypropylene and ethylene-propylene copolymer. The polymeric material may also contain functional groups such as carboxylates, esters, amines, aldehydes, alcohols, or halides, e.g., to provide sites for the attachment of chemical or biological moieties desirable to enhance the utility of the particles in chemical or biological analyses.

Methods for preparing nanoparticles or microparticles from polymers are well known in the art. For instance, proteins can be combined with non-protein polymers to form composite nanospheres or microspheres. In one embodiment, the particles are bioerodible synthetic or natural polymers. The term "bioerodible" or "biodegradable", as used herein refers to materials which are enzymatically, thermally, electric, ionic strength, pH, mechanically or chemically degraded or dissociate into simpler chemical species. Polysaccharides are examples of natural polymers. Synthetic polymers which degrade in *vivo* into innocuous products include poly(lactic acid) (PLA), poly(glycolic acid) (PGA) and co-polymers of PLA and PGA, polyorthoesters, polyanhydrides, polyphosphazene, polycaprolactone, polyhydroxybutyrate, blends and copolymers thereof. PLA, PGA and PLA/PGA copolymers are particularly useful for forming prolamine composite microspheres. PLA polymers are usually prepared from the cyclic esters of lactic acids. Both L(+) and D(-) forms of lactic acid can be used to prepare the PLA polymers, as well as the optically inactive DL-lactic acid mixture of mixtures of D(-) and L(+) lactic acids. Methods of preparing polylactides are well documented in the patent literature. The following U.S. patents describe in detail suitable polylactides, their properties and their preparation: 1,995,970 to Dorough; 2,703,316 to Schneider; 2,758,987 to Salzberg; 2,951,828 to Zeile; 2,676,945 to Higgins; and 2,683,136; 3,531,561 to Trehu. Thermoresponsive polymers include but are not limited to poly(*N*-isopropylacrylamide), hydroxypropylcellulose, poly(vinylcaprolactame), polyvinyl methyl ether, polyvinylmethylether and polyhydroxyethylmethacrylate.

In one embodiment, the particles are formed of a polysaccharide that can be easily degraded by an enzyme such as amylase. This would allow for the rapid removal of the particles prior to implantation.

The diameter of the particles may be similar to that of red blood cells so that single particles pass through the capillary bed. The particles may be from, for example, from about 0.01 µm to about 30 µm, from about 0.5 µm to about 20 µm, or about 5 µm to about 10 µm.

The particles may have any shape that is suitable for passage through the vasculature, including but not limited to, a sphere, elliptical shape, disk shape, donut shape, or star shaped, have concave or convex surfaces, and may have a smooth to non smooth surface.

The particles may have or be modified to have properties including but not limited to a hydrophilic surface to ensure easy passage, the ability of the surface to contract under pressure, such as a hydrogel or protein coating, the ability to be removed from the matrix either through degradation, mechanical (such as magnetic collection), or energy such that they break up into smaller pieces that can be successfully flushed from the matrix.

To ensure that the capillaries are formed with a sufficient diameter so that red blood cells will not be trapped after implantation, the particles may be added at the time of endothelial cell seeding or about 12 to 96 hours or up to weeks after cell seeding. For example, to expand the diameter of re-endothelialized vessels in otherwise decellularized grafts or in grafts recellularized with cells other than endothelial cells, the capillaries may be forced open by starting with a small particle size and then slowly increasing the particle size over hours/days until the desired size is able to perfuse through the matrix.

In one embodiment, the particles are formed of a thermoresponsive polymer that can be easily degraded or dissociated by a change in temperature. This would allow for the rapid removal of the particles prior to implantation.

In one embodiment, the particles are formed of a magnetic polymer and the addition of a magnetic source in the circulating solution enables the removal of circulating particles prior to implantation.

In one embodiment, the particles are removed from the re-endothelialized tissue or organ prior to transplantation. For biodegradable particles, a specific agent or condition would be added to degrade, dissolve, or digest the particles, followed by washing. The removal of particles may take place weeks/days or just before transplantation.

The normal concentration of red blood cells is about 3 to about 5 million/µL. Since the vasculature is about 10% of the overall tissue or organ void volume, the concentration of particles may be about 300-500,000 or up to 50 million per µL of tissue or organ perfusion (or void) volume.

To determine the efficacy of the particles in maintaining, enhancing or reducing a decrease in capillary vessel lumen diameter, the percent of particles that are recovered after perfusing the particles through the re-endothelialized matrix at physiological pressures is determined. For example, particles useful in the methods are those where, for instance, >50%, 60%, 70% or more of particles having a size that approximates the diameter of a blood cells, or have an average diameter of about 5 to 8 µm are recovered after being perfused through the tissue or organ, or blood is capable of being perfused through the tissue or organ, at physiological pressure with >50%, 60%, 70% return. In non-recelluarized organs, tissues, or portions of the majority of particles (5 to 8 µm) will reside in the interstitial space and not demonstrate significant return. In the re-endothelialized organs, tissues, or portions without particle treatment the majority of particles (e.g., those of about 5 µm to about 8 µm) will reside in the vasculature and do not clear the capillary beds.

## Claims

1. A method to maintain, reduce a decrease in or expand capillary vessel lumen diameter in a re-endothelialized extracellular matrix of a mammalian organ, tissue or portion thereof with an intact vascular bed, comprising:
(i) providing a decellularized extracellular matrix of a mammalian organ, tissue or portion thereof with an intact vascular bed and a population of endothelial cells or stem or progenitor cells capable of differentiation into endothelial cells, wherein the cells are not human embryonic stem cells; and
(ii) introducing an amount of the cells and a first aqueous solution comprising an amount of biocompatible microparticles to the decellularized extracellular matrix,
wherein the amount of the cells is effective to re-endothelialize the vasculature of the decellularized extracellular matrix and wherein the amount of the microparticles when circulated through the vasculature maintains, reduces a decrease or expands capillary vessel lumen diameter in the vasculature during or after re-endothelialization relative to a corresponding re-endothelialized decellularized extracellular matrix that lacks the microparticles.

2. The method of claim 1 wherein the microparticles maintain flow through the capillary beds.

3. The method of claim 1 or 2 wherein the microparticles are biodegradable, are not biodegradable, are magnetic, are spherical or elliptical, are deformable, or are formed of polymers, for example, formed of a naturally or non-naturally occurring polymer such as microparticles comprising alginate, polystyrene, polysaccharide, collagen, dextran, hyaluronic acid, glass, ceramic, metal, polylactic acid (PLA), poly-glutamic acid (PGA) or co-polymers of PLA and PGA (PLA/PGA), or microparticles comprising a hydrogel.

4. The method of any one of claims 1 to 3 wherein the microparticles comprise a hydrophilic surface or are modified to include carboxylates, esters, amines, aldehydes, alcohols, or halides or comprise a surface modification that binds a ligand.

5. The method of any one of claims 1 to 4 wherein the microparticles are formed of protein and non-protein polymers.

6. The method of any one of claims 1 to 5 wherein the average diameter of the microparticles is from about 0.5 µm to about 20 µm or about 5 to about 20 microns.

7. The method of any one of claims 1 to 6 wherein the solution is added after re-endothelialization.

8. The method of any one of claims 1 to 7 further comprising introducing a second aqueous solution comprising biocompatible microparticles having an average diameter that is at least 10% greater than the microparticles in the first solution and optionally further comprising introducing a third aqueous solution comprising biocompatible microparticles having an average diameter that is at least 10% greater than the microparticles in the second solution.

9. The method of any one of claims 1 to 8 wherein the first solution comprises about 300 to about 500,000 microparticles per µL.

10. The method of any one of claims 1 to 9 further comprising washing the vasculature with a solution that lacks the microparticles, for example, wherein the solution that lacks the nanoparticles or microparticles comprises an agent that degrades the microparticles, or wherein the solution that lacks the nanoparticles or microparticles is applied concurrent with an external factor that degrades or removes the microparticles including temperature, pH, ultrasound, light or electrical energy.

11. The method of any one of claims 1 to 10 wherein the organ is a heart, a pancreas, a bone, a liver, a kidney, or a lung.

12. The method of any one of claims 1 to 10 wherein the cells are obtained from iPS cells.

13. The method of any one of claims 1 to 12 wherein the population is introduced to the matrix either by injection or perfusion, or a combination thereof.

14. The method of any one of claims 1 to 13 wherein the population comprises primary cells, a plurality of different cell types or stem cells, wherein the cells are not human embryonic stem cells.

15. The method of any one of claims 1 to 14 wherein the cells and the perfusion decellularized organ or tissue are allogeneic or are xenogeneic.

## Patentansprüche

1. Verfahren zur Aufrechterhaltung, zur Verringerung der Abnahme oder zur Erweiterung des Durchmessers des Kapillargefäßlumens in einer re-endothelialisierten extrazellulären Matrix eines Säugetierorgans, eines Säugetiergewebes oder eines Teils davon mit einem intakten Gefäßbett, bei dem man
(i) eine dezellularisierte extrazelluläre Matrix eines Säugetierorgans, eines Säugetiergewebes oder eines Teils davon mit intaktem Gefäßbett sowie eine Population von endothelialen Zellen, Stammzellen oder Vorläuferzellen bereitstellt, die in der Lage sind, in endotheliale Zellen zu differenzieren, wobei es sich bei den Zellen nicht um humane embryonale Stammzellen handelt; und
(ii) eine Menge an Zellen und eine erste wässrige Lösung, die eine Menge an biokompatiblen Mikropartikeln enthält, in die dezellularisierte extrazelluläre Matrix einbringt;
wobei die Menge an Zellen wirksam ist, um das Gefäßsystem der dezellularisierten extrazellulären Matrix zu re-endothelialisieren, und wobei die Menge an Mikropartikeln bei Zirkulation durch die Gefäße während oder nach der Re-endothelialisierung im Vergleich zu einer entsprechenden re-endothelialisierten dezellularisierten extrazellulären Matrix, der die Mikropartikel fehlen, zur Aufrechterhaltung, zur Verringerung der Abnahme oder zur Erweiterung des Durchmessers des Kapillargefäßlumens führt.

2. Verfahren nach Anspruch 2, bei dem die Mikropartikel den Fluss durch das Kapillarbett aufrechterhalten.

3. Verfahren nach Anspruch 1 oder 2, bei dem die Mikropartikel biodegradierbar, nicht biodegradierbar, magnetisch, sphärisch oder elliptisch, deformierbar oder aus Polymeren aufgebaut sind, wie beispielsweise aus einem natürlich oder nicht-natürlich vorkommenden Polymer, wie beispielsweise Mikropartikel, die Alginat, Polystyrol, Polysaccharid, Kollagen, Dextran, Hyaluronsäure, Glas, Keramik, Metall, PolyMilchsäure (PLA), Poly-Glutaminsäure (PGA) oder Copolymere aus PLA und PGA (PLA/PGA) umfassen, oder Mikropartikel, die ein Hydrogel umfassen.

4. Verfahren nach einem der Ansprüche 1-3, wobei die Mikropartikel eine hydrophile Oberfläche umfassen, oder dahingehend modifiziert sind, dass sie Carboxylate, Ester, Amine, Aldehyde, Alkohole oder Halide umfassen oder eine Oberflächenmodifikation umfassen, die einen Liganden bindet.

5. Verfahren nach einem der Ansprüche 1-4, wobei die Mikropartikel aus Protein-Polymeren und Nicht-Protein-Polymeren aufgebaut sind.

6. Verfahren nach einem der Ansprüche 1-5, wobei der durchschnittliche Durchmesser der Mikropartikel von etwa 0,5 µm bis etwa 20 µm beträgt, oder von etwa 5 µm bis etwa 20 µm.

7. Verfahren nach einem der Ansprüche 1-6, wobei die Lösung nach der Re-endothelialisierung zugegeben wird.

8. Verfahren nach einem der Ansprüche 1-7, das ferner die Einbringung einer zweiten wässrigen Lösung umfasst, welche biokompatible Mikropartikel umfasst, die einen durchschnittlichen Durchmesser aufweisen, der mindestens 10 % größer ist als der der Mikropartikel in der ersten Lösung, und wobei das Verfahren ferner optional die Einbringung einer dritten wässrigen Lösung umfasst, welche biokompatible Mikropartikel umfasst, die einen durchschnittlichen Durchmesser aufweisen, der mindestens 10 % größer ist als der der Mikropartikel in der zweiten Lösung.

9. Verfahren nach einem der Ansprüche 1-8, wobei die erste Lösung etwa 300 bis etwa 500.000 Mikropartikel pro µL umfasst.

10. Verfahren nach einem der Ansprüche 1-9, bei dem man ferner das Gefäßsystem mit einer Lösung wäscht, die frei von Mikropartikeln ist, wobei die Lösung, der die Nanopartikel oder Mikropartikel fehlen, beispielsweise ein Mittel umfasst, das die Mikropartikel abbaut, oder wobei die Lösung, der die Nanopartikel oder Mikropartikel fehlen, zusammen mit einem externen Faktor angewendet wird, welcher die Mikropartikel abbaut oder entfernt, einschließlich Temperatur, pH, Ultraschall, Licht oder elektrische Energie.

11. Verfahren nach einem der Ansprüche 1-10, wobei das Organ ein Herz, eine Bauchspeicheldrüse, ein Knochen, eine Leber, eine Niere oder eine Lunge ist.

12. Verfahren nach einem der Ansprüche 1-10, wobei die Zellen von iPS-Zellen erhalten werden.

13. Verfahren nach einem der Ansprüche 1-12, wobei die Population entweder durch Injektion, Perfusion oder durch eine Kombination davon in die Matrix eingebracht wird.

14. Verfahren nach einem der Ansprüche 1-13, wobei die Population Primärzellen, eine Vielzahl von verschiedenen Zell-Typen oder Stammzellen umfasst, wobei es sich bei den Zellen nicht um humane embryonale Stammzellen handelt.

15. Verfahren nach einem der Ansprüche 1-14, wobei die Zellen und das mittels Perfusion dezellularisierte Organ oder Gewebe allogen oder xenogen sind.

## Revendications

1. Procédé pour maintenir, réduire une diminution du ou augmenter le diamètre de la lumière de vaisseaux capillaires dans une matrice extracellulaire ré-endothélialisée d'un organe mammalien, d'un tissu mammalien ou d'une partie de ceux-ci comportant un lit vasculaire intact, comprenant :
(i) le fait de fournir une matrice extracellulaire décellularisée d'un organe mammalien, d'un tissu mammalien ou d'une partie de ceux-ci comportant un lit vasculaire intact et une population de cellules endothéliales ou de cellules souches ou progénitrices capables de différenciation en cellules endothéliales, les cellules n'étant pas des cellules souches embryonnaires humaines ; et
(ii) le fait d'introduire une quantité des cellules et une première solution aqueuse comprenant une quantité de microparticules biocompatibles dans la matrice extracellulaire décellularisée,
dans lequel la quantité des cellules est efficace pour ré-endothélialiser le système vasculaire de la matrice extracellulaire décellularisée et dans lequel la quantité des microparticules, lorsqu'elles sont mises en circulation dans le système vasculaire, maintient, réduit une diminution du ou augmente le diamètre de la lumière des vaisseaux capillaires dans le système vasculaire pendant ou après la ré-endothélialisation par rapport à une matrice extracellulaire décellularisée ré-endothélialisée correspondante qui est dépourvue des microparticules.

2. Procédé selon la revendication 1, dans lequel les microparticules maintiennent la circulation dans les lits capillaires.

3. Procédé selon la revendication 1 ou 2, dans lequel les microparticules sont biodégradables, ne sont pas biodégradables, sont magnétiques, sont sphériques ou elliptiques, ou sont constituées de polymères, par exemple, constituées d'un polymère existant dans la nature ou n'existant pas dans la nature, telles que des microparticules comprenant de l'alginate, du polystyrène, un polysaccharide, du collagène, du dextrane, de l'acide hyaluronique, du verre, de la céramique, du métal, du poly(acide lactique) (PLA), du poly(acide glutamique) (PGA) ou des copolymères de PLA et PGA (PLA/PGA), ou des microparticules comprenant un hydrogel.

4. Procédé selon l'une quelconque des revendications 1 à 3, dans lequel les microparticules comprennent une surface hydrophile, ou sont modifiées pour inclure des carboxylates, des esters, des amines, des aldéhydes, des alcools ou des halogénures ou comprennent une modification de la surface qui se lie à un ligand.

5. Procédé selon l'une quelconque des revendications 1 à 4, dans lequel les microparticules sont constituées de polymères protéiques et de polymères non protéiques.

6. Procédé selon l'une quelconque des revendications 1 à 5, dans lequel le diamètre moyen des microparticules vaut d'environ 0,5 µm à environ 20 µm ou d'environ 5 à environ 20 micromètres.

7. Procédé selon l'une quelconque des revendications 1 à 6, dans lequel on ajoute la solution après la ré-endothélialisation.

8. Procédé selon l'une quelconque des revendications 1 à 7, comprenant encore le fait d'introduire une deuxième solution aqueuse comprenant des microparticules biocompatibles ayant un diamètre moyen qui est supérieur d'au moins 10 % à celui des microparticules dans la première solution et en option comprenant encore le fait d'introduire une troisième solution aqueuse comprenant des microparticules biocompatibles ayant un diamètre moyen qui est supérieur d'au moins 10 % à celui des microparticules dans la deuxième solution.

9. Procédé selon l'une quelconque des revendications 1 à 8, dans lequel la première solution comprend environ 300 à environ 500 000 microparticules par µL.

10. Procédé selon l'une quelconque des revendications 1 à 9, comprenant encore le fait de laver le système vasculaire avec une solution qui est dépourvue des microparticules, par exemple, la solution qui est dépourvue des nanoparticules ou microparticules comprenant un agent qui dégrade les microparticules, ou la solution qui est dépourvue des nanoparticules ou microparticules étant appliquée conjointement avec un facteur externe qui dégrade ou élimine les microparticules, incluant la température, le pH, les ultrasons, la lumière ou l'énergie électrique.

11. Procédé selon l'une quelconque des revendications 1 à 10, dans lequel l'organe est un coeur, un pancréas, un os, un foie, un rein ou un poumon.

12. Procédé selon l'une quelconque des revendications 1 à 10, dans lequel les cellules sont obtenues à partir de cellules SPi.

13. Procédé selon l'une quelconque des revendications 1 à 12, dans lequel on introduit la population dans la matrice soit par injection soit par perfusion, ou par une combinaison des deux.

14. Procédé selon l'une quelconque des revendications 1 à 13, dans lequel la population comprend des cellules primaires, une pluralité de types cellulaires différents ou des cellules souches, les cellules n'étant pas des cellules souches embryonnaires humaines.

15. Procédé selon l'une quelconque des revendications 1 à 14, dans lequel les cellules et l'organe ou le tissu décellularisé perfusé sont allogéniques ou sont xénogéniques.
